# EUROPEAN PATENT SPECIFICATION

(11) **EP 4 656 171 B1**
(45) Date of publication and mention of the grant of the patent: **23.09.2026**
(21) Application number: 23930089.0
(22) Date of filing: 13.12.2023
(51) Int. Cl.: A61H 1/00, A61H 23/00, A61G 7/043, A61M 21/02

(54) **PASSIVE RELAXATION APPARATUS**
PASSIVE ENTSPANNUNGSVORRICHTUNG
APPAREIL DE RELAXATION PASSIVE

(30) Priority: 28.03.2023 CN 202310333521
(43) Date of publication of application: 03.12.2025
(73) Proprietor: Keeson Technology Corporation Limited, Jiaxing, Zhejiang 314016 (CN)
(72) Inventor: SHAN, Huafeng, Jiaxing, Zhejiang 314016 (CN); ZHANG, Jianwei, Jiaxing, Zhejiang 314016 (CN); GE, Hewen, Jiaxing, Zhejiang 314016 (CN); XU, Zherui, Jiaxing, Zhejiang 314016 (CN); XU, Jiaxin, Jiaxing, Zhejiang 314016 (CN)
(74) Representative: Santarelli
(86) International application number: PCT/CN2023/138477
(87) International publication number: WO 2024/198524

(56) References cited:
- EP-A1- 2 168 458
- EP-A1- 2 168 458
- EP-A1- 2 168 550
- EP-A1- 2 198 751
- CN-A- 1 178 667
- CN-A- 1 178 667
- CN-A- 110 339 453
- CN-A- 112 870 526
- CN-A- 112 870 526
- CN-A- 113 908 004
- CN-A- 116 473 789
- JP-A- 2001 149 164
- JP-A- 2004 073 550
- KR-A- 20210 123 832
- US-B1- 8 707 477

## Description

### Technical Field

The present invention relates to the field of sleep-assistance technolonies and in particular, to a passive relaxation apparatus.

### Background of the Invention

In modern society, with a fast pace of life and high work pressure, people are prone to insomnia. Although insomnia is not a critical illness, it hinders people's normal life, work, study, and health, and can exacerbate or induce conditions such as palpitations, chest discomfort, dizziness, headaches, and strokes. Persistent insomnia causes long-term suffering to patients and may even lead to dependence on sleeping pills, while long-term use of sleeping pills can cause iatrogenic diseases.

Currently, electric beds can be used to enhance the comfort of the human body during rest. Electric beds in the prior art comprise: a back bed panel, a waist bed panel, a thigh bed panel, and a calf bed panel. The waist bed panel remains stationary, and the electric bed is positioned into specific configurations by rotating the back bed panel, thigh bed panel, and calf bed panel. Some electric beds are also equipped with an additional head bed panel on the back bed panel, which can be further rotated relative to the back bed panel. Adjusting the posture of the electric bed can improve human comfort.

Chinese Patent Application No. CN112870526 discloses a sleep-aid control method, which includes a relaxation phase for controlling the electric bed to execute a human body relaxation mode to perform relaxation massage on human muscles; and a sleep-aid phase for controlling the electric bed to execute a postural sleep-aid mode to promote intracranial venous return in the human body. Here, the human body relaxation mode involves massaging specific parts of the human body in a zero-gravity mode, and the postural sleep-aid mode involves controlling the bed panels to move to specific angles to improve intracranial venous return in the human body. The patent document further discloses a sleep-aid control device, including a relaxation module configured to, in a zero-gravity posture mode, control bed panels corresponding to the user's back and thigh regions to rotate upward, such that the user's back forms an angle of 10 to 30 degrees relative to the horizontal plane, and the user's thighs form an angle of 20 to 50 degrees relative to the horizontal plane; and a sleep-aid module configured to, in a postural sleep-aid mode, control the bed panel of the electric bed corresponding to the upper body to rotate, so that the head forms an angle of 5 to 30 degrees relative to the horizontal plane, thereby promoting intracranial venous return. The rotational speed of the bed panel in the relaxation phase is greater than or equal to the rotational speed thereof in the sleep-aid phase.

However, the devices and methods in the prior art are unable to provide deep relaxation to the human body and do not fully utilize the functions of electric beds. Other prior art documents disclosing adjustable head and foot support portions include US8707477B1, EP2168458A1, CN1178667A and JP2004073550A.

### Summary of the Invention

One of the objectives of this application is to overcome one of the deficiencies in the prior art by providing a passive relaxation apparatus to address the issue of enabling deep relaxation of the human body. The invention is set out in the appended claims.

### Summary of the disclosure

For this, some embodiments of this application provide a passive relaxation apparatus which includes a human body support unit and at least one position adjustment component. The initial position of the human body support unit is horizontal and comprises a head support portion, a waist support portion, and a foot portion corresponding to the head, waist, and foot positions of the human body, respectively. The position adjustment component is configured to change the inclination angle of at least one of these portions of the human body support unit according to instructions. The waist support portion further includes a first vibration component. The control module comprises a controller and a relaxation execution unit. The controller is configured to generate sequential first control instruction and second control instruction according to first-stage relaxation mode data. The relaxation execution unit is configured to drive the at least one position adjustment component according to the first control instruction to change the inclination angle of at least one portion of the human body support unit and, during this process, activate the first vibration component for a first time period; and to drive the position adjustment component to alternately elevate the head portion and the foot portion according to the second control instruction to form a swinging motion and perform this swinging motion multiple times.

Wherein, changing the inclination angle of at least one portion of the human body support unit in response to the first control instruction includes driving the head support portion from a horizontal position to a position at a 15-degree angle to the horizontal, driving the foot support portion from a horizontal position to a position at a 35-degree angle to the horizontal, activating the first vibration component, and maintaining it for the first time period, during which the first vibration component vibrates at a first intensity, for example, level four, for the first time period, such as 50 seconds.

Wherein, the swinging motion includes, during its first time period, driving the head support portion from a horizontal position to a position at a 15-degree angle to the horizontal to elevate the head support portion while lowering the foot support portion to a position at a 15-degree angle to the horizontal, and during its second time period, driving the foot support portion from a position at a 15-degree angle to the horizontal to a position at a 35-degree angle to the horizontal while driving the head support portion from a position at a 15-degree angle to the horizontal to a position at a 2-degree angle to the horizontal.

Wherein, during each swinging motion, the movement rates of the head support portion and the foot support portion are the same. Based on this, in some embodiments, the movement speeds of the head support portion during the swinging motion are all the same, and the movement speeds of the foot support portion during the swinging motion are all the same. In other embodiments, the movement speed of the head support portion during the swinging motion gradually decreases, and the movement speed of the foot support portion during the swinging motion gradually decreases.

Wherein, the swinging motion is performed four times.

In some embodiments, the human body support unit further includes a second vibration component in the foot support portion. The first vibration component, the second vibration component, and the position adjustment component are configured to be driven according to instructions from the second-stage relaxation mode.

Wherein, the actions specified in the instructions of the second-stage relaxation mode include maintaining the head support portion and the foot support portion horizontal, activating the first vibration component and the second vibration component, and performing a first control instruction for a full-body vibration massage relaxation with continuous vibrations at a frequency of 28 Hz to 52 Hz. Here, the first vibration component is configured to vibrate continuously for a second time period, such as 3 minutes, at a second intensity, for example, level four, and the second vibration component is configured to vibrate continuously for the second time period, such as 3 minutes, at a third intensity, for example, level eight, for full-body vibration massage relaxation.

Wherein, the second-stage relaxation mode may include one of the first vibration mode, the second vibration mode, the third vibration mode, and the fourth vibration mode alone, or any combination of two in any order, any combination of three in any order, or all four in any order. The first vibration mode includes vibrating at a frequency of 28 Hz, which can effectively promote blood circulation; the second vibration mode includes vibrating at a frequency of 40 Hz, which can help the user relax physically and mentally, relieve depression, and thus improve sleep quality; the third vibration mode includes vibrating at a frequency of 44 Hz, which is more beneficial for muscle relaxation, eliminating muscle fatigue, restoring muscle strength, and improving muscle tendon elasticity and stiffness; the fourth vibration mode includes vibrating at a frequency of 52 Hz, which can help reduce lower back pain.

In some embodiments, the control module further includes a mode selection unit configured to receive the selection of any one vibration mode or any combination thereof in the second-stage relaxation mode of the passive relaxation apparatus. For example, the second-stage relaxation mode can be selected to sequentially execute the control instructions corresponding to the above-mentioned first vibration mode, second vibration mode, third vibration mode, and fourth vibration mode.

In some embodiments, the mode selection unit is configured to determine the vibration mode in the second-stage relaxation mode according to user input.

The second-stage relaxation mode further includes a fourth control instruction. While executing the fourth control instruction, the head support portion is elevated to 15 degrees, the foot support portion is placed horizontally, and the second vibration component of the foot support portion is activated at a fourth intensity, for example, level four, for a third time period, such as 155 seconds. Then, the head support portion is placed horizontally from 15 degrees, and the foot support portion is maintained horizontal.

In some embodiments, the first vibration component, the second vibration component, and the position adjustment component are configured to be driven according to instructions from the third-stage relaxation mode.

Wherein the instructions of the third-stage relaxation mode include driving the head support portion to be elevated to a position at a 15-degree angle to the horizontal, driving the foot support portion to be elevated to a position at a 35-degree angle to the horizontal, maintaining this position for a fourth time period, such as 160 seconds; driving the foot support portion to descend to the horizontal and maintaining this position for a fifth time period, such as 235 seconds; and finally placing the head support portion horizontal.

In some embodiments according to this application, the apparatus further include a communication unit that receives the first-stage relaxation mode, the second-stage relaxation mode, and the third-stage relaxation mode sent through a cloud server, transmits parameter adjustment instructions, and sends them to the control module.

Some embodiments of this application also provide a control method for a passive relaxation apparatus, which includes executing one or more of the above-mentioned first-stage relaxation mode, second-stage relaxation mode, and third-stage relaxation mode according to the above steps.

This application also provides a computer device, comprising: a memory for storing computer program instructions and a processor for executing the computer program instructions, wherein when the computer program instructions are executed by the processor, the computer device is triggered to execute the method of the present application, which is presented here for illustration purposes and is not forming part of the invention.

The passive relaxation apparatus and method (not part of the invention) provided by this application perform moderate stretching and vibration massage on the human body during the relaxation phase, relieve tense muscles, eliminate muscle fatigue, improve human comfort, and enable the human body to enter a state of deep relaxation.

### Brief Description of the Drawings

By reading the detailed description of non-limiting embodiments with reference to the following accompanying drawings, other features, objectives, and advantages of the present invention will become more apparent:
Figure 1 is a schematic figure of the horizontal posture of a passive relaxation apparatus according to an embodiment of the present invention;
Figure 2 is a schematic figure showing the changed positions of the head support portion and foot portion of the passive relaxation apparatus in Figure 1;
Figure 3 is a schematic figure of the horizontal posture of another passive relaxation apparatus according to an embodiment of the present invention;
Figure 4 is a schematic figure showing the changed positions of the head support portion and foot portion of the passive relaxation apparatus in Figure 3;
Figure 5 is a structural figure of the control module of the passive relaxation apparatus according to an embodiment of the present application;
Figure 6 is a control flowchart of the control module executing the three stages' relaxation mode according to an embodiment of the present application;
Figure 7 is a relationship figure illustrating the composition of the first-stage relaxation mode according to an embodiment of the present application;
Figure 8 is a flowchart of the second-stage relaxation mode according to an embodiment of the present application;
Figure 9 is a flowchart of the third-stage relaxation mode according to an embodiment of the present application;
Figure 10 is a flowchart of the steps for outputting a report according to an embodiment of the present application;
Figure 11A is a stress information graph of a test user before using the apparatus in the embodiment of the present application, according to the verification example of the present application;
Figure 11B is a heart rate information graph of the test user before using the apparatus in the embodiment of the present application, according to the verification example of the present application;
Figure 11C is a respiratory rate graph of the test user before using the apparatus in the embodiment of the present application, according to the verification example of the present application;
Figure 12A is a stress information graph of the test user after using the apparatus in the embodiment of the present application once, according to the verification example of the present application;
Figure 12B is a heart rate information graph of the test user after using the apparatus in the embodiment of the present application once, according to the verification example of the present application;
Figure 12C is a respiratory rate information graph of the test user after using the apparatus in the embodiment of the present application once, according to the verification example of the present application;
Figure 13 is a comparative graph of curve graphs from the latency period to the N1 sleep stage according to the verification example of the present application;
Figure 14 is another comparative graph of curve graphs from the latency period to the N1 sleep stage according to the verification example of the present application;
Figure 15 is a comparative graph of line graphs depicting sleep duration according to the verification example of the present application;
Figure 16 is a comparative graph of box plots depicting sleep duration according to the verification example of the present application.

### Detailed Description of Embodiments

The specific implementation methods of this application will be described in detail below in conjunction with the accompanying figures, tables, and lists of the specification.

The specific structural and functional details disclosed herein are merely representative and are intended for the purpose of describing exemplary embodiments of this application. However, this application can be implemented in many alternative forms and should not be construed as being limited solely to the embodiments described herein.

It should be understood that although terms such as "first," "second," etc., may be used herein to describe various units, these units should not be limited by these terms. These terms are used merely to distinguish one unit from another. For example, without departing from the scope of the exemplary embodiments, the first unit may be referred to as the second unit, and similarly, the second unit may be referred to as the first unit. The term "and/or" used herein includes any and all combinations of one or more of the listed associated items.

The terms used herein are solely for the purpose of describing specific embodiments and are not intended to limit the exemplary embodiments. Unless explicitly stated otherwise in the context, the singular forms "a", "an" are also intended to include the plural forms. It should also be understood that the terms "include" and/or "comprise" specify the presence of stated features, integers, steps, operations, units, and/or components, but do not preclude the presence or addition of one or more other features, integers, steps, operations, units, components, and/or groups thereof.

It should also be mentioned that in some alternative implementations, the mentioned functions/actions may occur in a different order than that indicated in the accompanying drawings. For example, depending on the involved functions/actions, two consecutively shown figures may actually be executed substantially simultaneously or sometimes in reverse order.

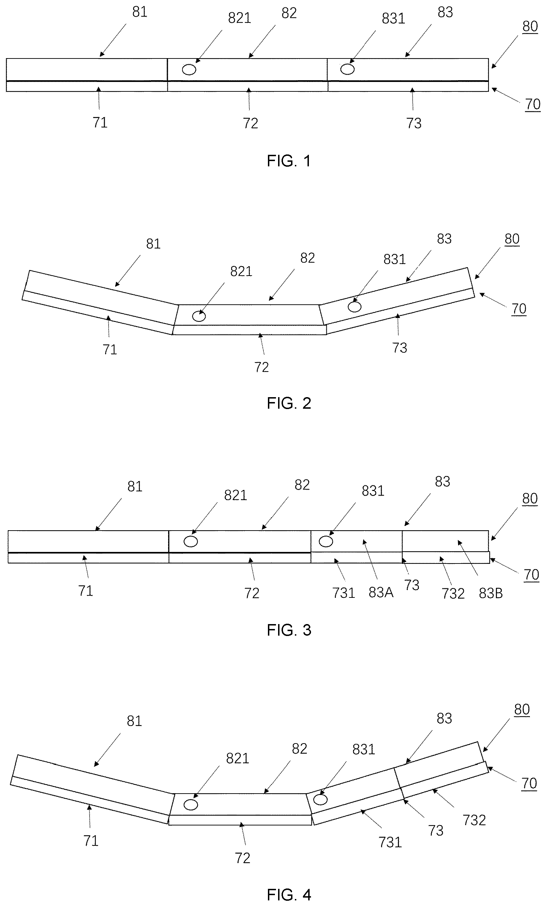

Referring to Figure 1, some embodiments of this application provide a passive relaxation apparatus, including a human body bearing unit 80 and at least one position adjustment component 70. The initial position of the human body bearing unit 80 is horizontal and includes a head portion 81, a waist support portion 82, and a foot support portion 83 corresponding to the head, waist, and foot positions of the human body, respectively. The position adjustment component 70 is configured to change the relative horizontal inclination angles of these portions of the human body bearing unit according to instructions, such as including a head adjustment component 71, a waist adjustment component 72, and a foot adjustment component 73. The passive relaxation apparatus also includes a controller unit 85 configured to read relaxation mode data from a storage unit 85 and generate control instructions according to the relaxation mode through a relaxation execution unit 841 to adjust the position adjustment component 70 and implement the relaxation mode. In the human body bearing unit, the back support portion 82 also includes a first vibration component 821, such as a first vibration generator.

The head portion 81, waist support portion 82, and foot support portion 83 may be separately arranged or coupled together via hinges.

Figure 2 shows a schematic figure of the human body bearing unit 80 and position adjustment component 70 of the passive relaxation apparatus in a relaxation mode. It can be seen that the head portion 81 is lifted by the head adjustment component 71 to change its angle, and the foot support portion 83 is lifted by the foot adjustment component 73 to change its angle.

Figure 3 is a structural schematic figure of another passive relaxation apparatus provided in some embodiments of this application. The main structural difference between the passive relaxation apparatus in Figure 3 and that in Figure 1 is that the foot support portion 83 in Figure 3 includes a first foot support portion 831 and a second foot support portion 832 that are pivotally connected to each other, and the corresponding foot adjustment component 73 includes a first foot adjustment component 731 for changing the inclination angle of the first foot support portion 831 and a second foot adjustment component 732 for changing the inclination angle of the second support foot support portion 832. In the human body bearing unit, the back support portion 82 also includes a first vibration component 821, such as a first vibration generator. Figure 4 is a schematic figure of the passive relaxation apparatus in Figure 3 in a relaxation mode. As shown in Figure 4, the first foot adjustment component 731 and the second foot adjustment component 732 can drive the first foot support portion 831 and the second foot support portion 832 to the same inclination angle. Of course, it should be understood that the first foot adjustment component 731 and the second foot adjustment component 732 can also drive the first foot support portion 831 and the second foot support portion 832 to different inclination angles according to the relaxation mode.

In other embodiments of this application, which are not part of the claimed invention, the human body support unit 80 may only include the head support portion 81 and the foot support portion 83. Correspondingly, the position adjustment component only includes the head adjustment component 71 and the foot adjustment component 73.

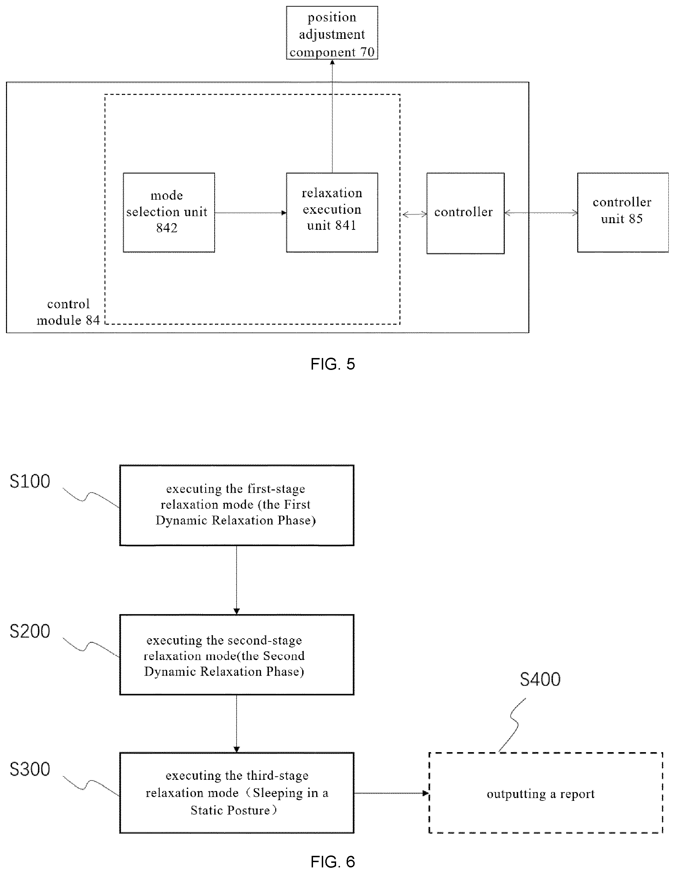

In the embodiments of this application, the relaxation mode may include a first-stage relaxation mode, a second-stage relaxation mode, and a third-stage relaxation mode executed sequentially. As shown in Figure 6, the entire passive relaxation process may include sequentially executing the first-stage relaxation mode, step S100, executing the second-stage relaxation mode, step S200, and executing the third-stage relaxation mode, step S300. The control of the passive relaxation apparatus under each stage of the relaxation mode will be described in detail below.

In the embodiments of this application, the human body bearing unit 80 may be the bed board of an electric bed. For example, the head support portion 81 may be the head bed board of the electric bed that can pivot relative to the waist bed board, the waist support portion 82 may be the fixed waist bed board of the electric bed, and the foot support portion 83 may be the foot bed board of the electric bed that can pivot relative to the waist bed board. Correspondingly, the position adjustment component 70 may be the cylinder unit of the electric bed. The human body bearing unit 80 may also be a segmented mattress. For example, the head support portion 81 may be the head mattress portion of the segmented mattress that can pivot relative to the waist mattress portion, the waist support portion 82 may be the waist mattress portion, and the foot support portion 83 may be the foot mattress portion that can pivot relative to the waist mattress portion.

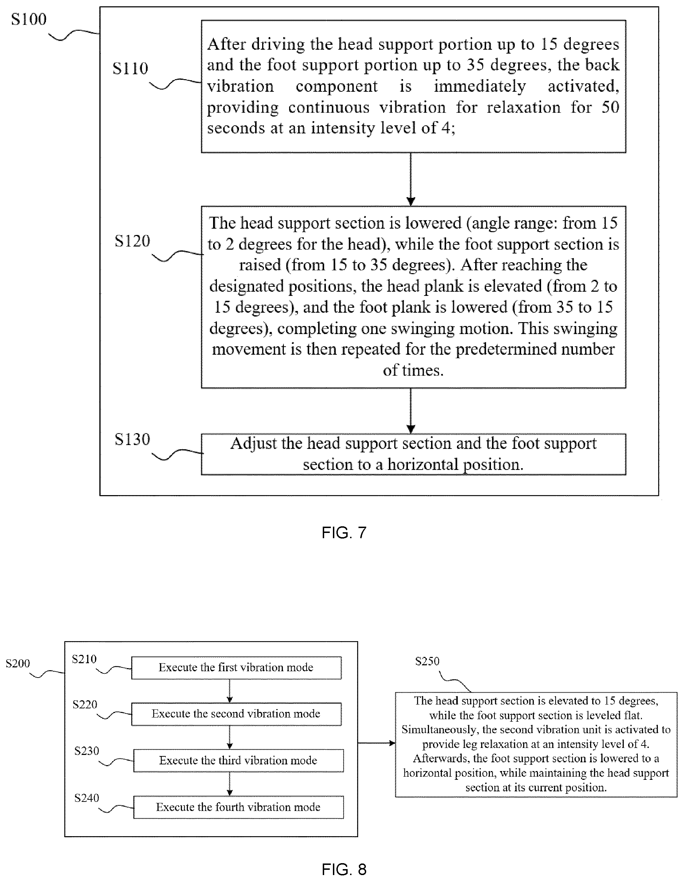

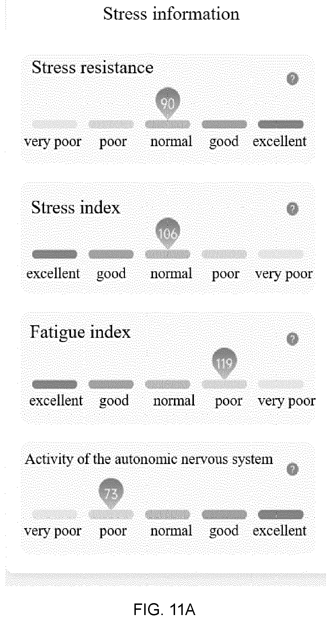

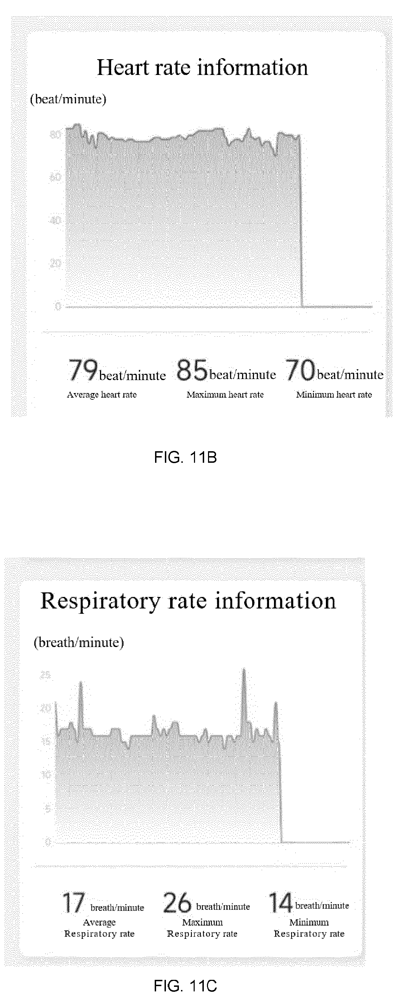

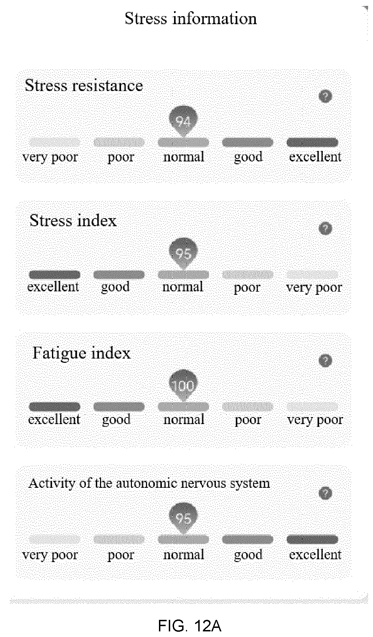

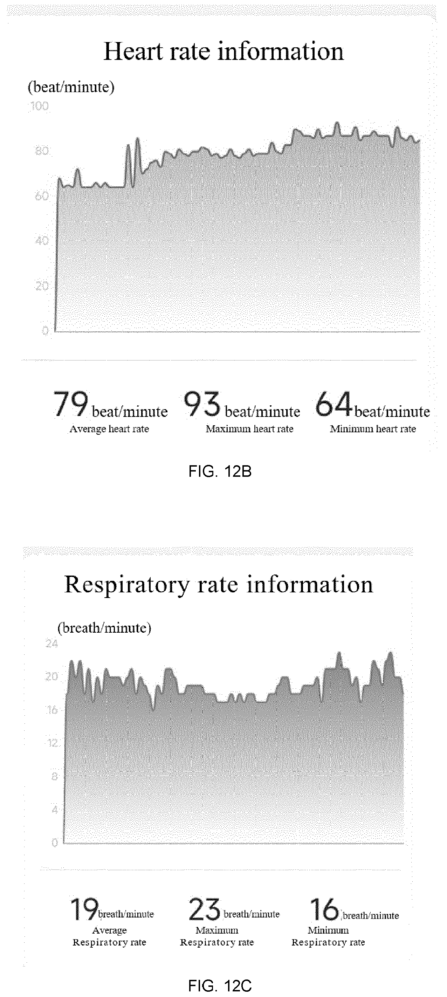

As shown in Figure 7, the first-stage relaxation mode may include a first relaxation program part S110 based on a first control instruction and a second relaxation program part S120 based on a second control instruction. In the first relaxation program part S110, the head support portion 81 of the human body bearing unit is driven to rise to a position at a 15-degree angle to the horizontal, the foot support portion 83 is driven to rise to a position at a 30-degree angle to the horizontal, the first vibration component 821 is turned on and maintained for a first time period, during which the first vibration component vibrates at level 4 intensity for 50 seconds. The second relaxation program part S120 includes alternately driving the head portion and the foot portion to rise by the position adjustment component, specifically including driving the head portion 81 from a 2-degree position to a position at a 15-degree angle to the horizontal while lowering the foot support portion 83 from a position at a 35-degree angle to the horizontal to a position at a 15-degree angle to the horizontal, and then executing the reverse process, i.e., driving the foot support portion from a position at a 15-degree angle to the horizontal to a position at a 35-degree angle to the horizontal while driving the head support portion from a position at a 15-degree angle to the horizontal to a position at a 2-degree angle to the horizontal, forming a swinging motion. This process can be continuously executed four times. The duration of the four swinging motions helps to maximize the reduction of the user's stress index. The stress index of the user after adjustment by the device in this application is shown in Figures 11A and 12A, the heart rate information is shown in Figures 11B and 12B, and the respiratory rate is shown in Figures 11C and 12C. As can be seen from the figures, after adjustment by the device in the embodiments of this application, the activity of the user's autonomic nervous system significantly increases, and the fatigue index and stress index significantly decrease. The heart rate gradually stabilizes, and the respiratory rate gradually approaches stability.

**In the figures,** the fatigue index is a quantitative indicator of the degree of fatigue. It is related to indicators such as **TP,** LF, and the stress index in frequency domain analysis. The lower the activity of the autonomic nervous system and the heavier the stress borne, the higher the fatigue index. The higher the degree of physical and mental fatigue caused by stress, the more likely it is to experience abnormal somatic symptoms such as drowsiness, weakness, and muscle weakness. Stress resistance refers to the body's ability to adapt to environmental changes or various stress stimuli and observe its ability to maintain a stable internal environment. It reflects the overall standard deviation SDNN parameter (an indicator of the degree of heart rate variability) in time domain analysis. A decrease in HRV means a decrease in the complexity of heart rate motility variation, indicating that the body has a poor ability to adapt to environmental changes, a decrease in stress resistance, a decline in the body's ability to maintain dynamic balance (stability), and a high likelihood of dysfunction and various diseases once subjected to stress. The stress index is a quantitative indicator of the current stress borne. The degree of stress can be observed through various parameters in the time domain such as HR (heart rate) and HRV. The higher the stress index, the heavier the fatigue felt by the body, and symptoms such as headache, insomnia, muscle soreness, and palpitations are more likely to occur, accompanied by mental and emotional instability. Normal range: 50-110 (the smaller the stress index, the less stress is borne, and the better the physical condition). The activity of the autonomic nervous system reflects the overall activity level of the autonomic nervous system and can assess the regulatory ability of the autonomic nervous system; the higher the activity, the better the self-regulatory ability. It is related to the total energy (TP) in frequency domain analysis and has a similar representative meaning to the heart rate variability standard deviation (SDNN) parameter in time domain analysis. In cases of chronic stress or disease, the regulatory ability of the autonomic nervous system is low, and the total energy (TP) is significantly lower than the normal range. Normal range: 90-150.

It should be understood that during each swinging motion, such as the first motion, 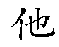 the second motion, the third motion, and the fourth swinging motion, the movement rates of the head support portion and the foot support portion are the same. On this basis, in some embodiments, the movement speeds of the head support portion during the four swinging motions are the same, and the movement speeds of the foot support portion during the four swinging motions are also the same; while in other embodiments, the movement speed of the head support portion during the four swinging motions gradually decreases, for example, by 1/2 each time, and the movement speed of the foot support portion during the swinging motions also gradually decreases, for example, by 1/2 each time. The main function of the swinging motion is to lull the user to sleep, and gradually decreasing the speed of the swinging motion helps to produce a lulling effect.

In some embodiments, the foot support portion 83 also includes a second vibration component 831, such as a second vibration generator, where the first vibration component 821, the second vibration component 831, and the position adjustment component 70 are configured to be further driven according to the instructions of the second-stage relaxation mode, step S200.

As shown in Figure 8, after the instructions for the second-stage relaxation mode start to be executed, the actions corresponding to these instructions include any sequential combination of one, two, three, or four of the following driving methods. For example, the driving methods may include keeping the head support portion horizontal, turning on the first vibration component 821 to operate at a vibration frequency of 28 Hz and a vibration intensity of level 4, and the second vibration component 831 to operate at a vibration frequency of 28 Hz and a vibration intensity of level 10, all maintained for three minutes, step S210; keeping the head support portion horizontal, turning on the first vibration component 821 to operate at a vibration frequency of 40 Hz and a vibration intensity of level 4, and the second vibration component 831 to operate at a vibration frequency of 40 Hz and a vibration intensity of level 10, all maintained for three minutes, step S220; turning on the first vibration component 821 to operate at a vibration frequency of 44 Hz and a vibration intensity of level 4, and the second vibration component 831 to operate at a vibration frequency of 44 Hz and a vibration intensity of level 10, all maintained for three minutes, step S230; and turning on the first vibration component 821 to operate at a vibration frequency of 52 Hz and a vibration intensity of level 4, and the second vibration component 831 to operate at a vibration frequency of 52 Hz and a vibration intensity of level 10, all maintained for three minutes, step S240. After the above vibration relaxation program is completed, the head support portion 81 can be driven to rise to 15 degrees, the foot support portion 83 can be placed horizontally, and the second vibration component can be turned on at an intensity of level 4 for 155 seconds while keeping the foot support portion 83 horizontal and driving the head support portion 81 to the horizontal position, step S250.

In some embodiments, the first vibration component 821, the second vibration component 831, and the position adjustment component 70 are further configured to be driven according to the instructions of the third-stage relaxation mode, step S300.

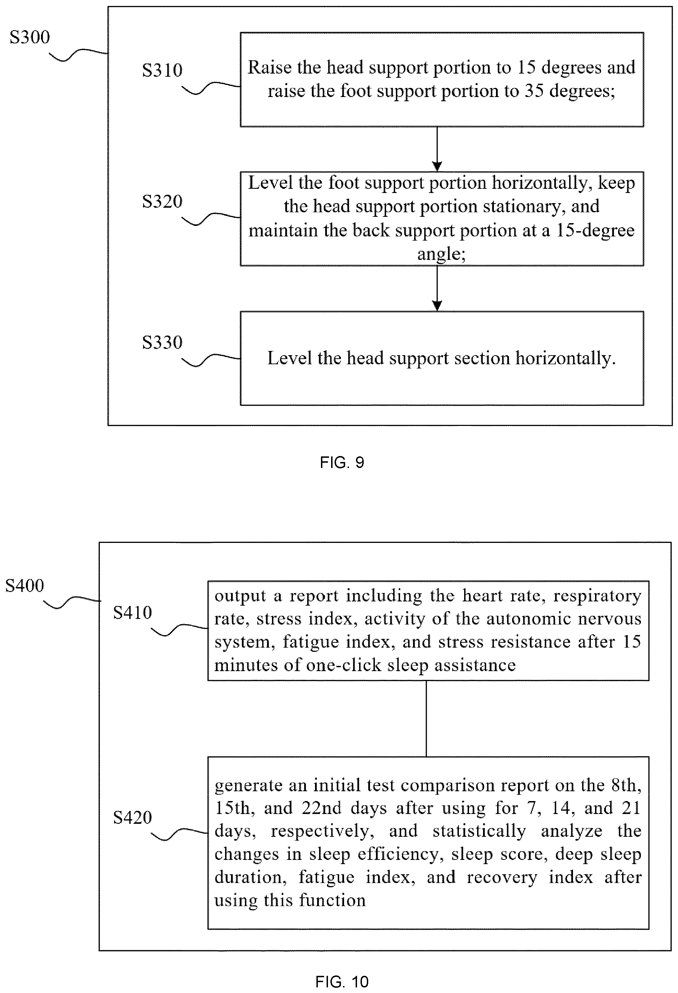

As shown in Figure 9, after the instructions for the third-stage relaxation mode start to be executed, the corresponding actions include driving the head support portion to rise to 15 degrees and the foot support portion to rise to 35 degrees , and maintain the position for 160 seconds, step S310; flattening the foot support portion while keeping the head support portion stationary and the back support portion at 15 degrees for 235 seconds, step S320; and flattening the head support portion, step S330.

As shown in Figure 5, in some embodiments of this application, the control module of the passive relaxation apparatus may also include a mode selection unit 842 configured to receive the selection of the first-stage relaxation mode of the passive relaxation apparatus and change the relaxation execution unit to generate the instructions for the first-stage relaxation mode according to the selection result.

Some embodiments of this application also include a communication unit that receives the first-stage relaxation mode, second-stage relaxation mode, and third-stage relaxation mode sent through a cloud server and transmits parameter adjustment instructions, and sends them to the control module for storage in the storage unit for subsequent retrieval.

The first-stage relaxation mode, second-stage relaxation mode, and third-stage relaxation mode can achieve individual effects when performed separately, but when combined in sequence, they can produce unexpected comprehensive effects.

The first relaxation program part S110 of the first-stage relaxation mode first puts the body into a natural relaxation state through a zero-gravity and massage mode, with the angle between the upper torso and the thighs being 128° (±7°), in a neutral body posture. At the same time, when the body is in a zero-gravity state, i.e., the most relaxed reclining state, the massage is a deep massage, and the massage effect is also the best. During the vibration process, static stretching has a significant effect on alleviating muscle damage and fatigue recovery. The second relaxation program part S120 induces drowsiness through a swinging lulling function, promoting relaxation and thus regulating sleep. At the same time, the swinging motion can positively increase the duration of spindle waves and the deep sleep stage, thereby improving sleep quality.

In the second-stage relaxation mode, vibration at a frequency of 28 Hz can effectively promote blood circulation; vibration at a frequency of 40 Hz can help the user relax mentally and relieve depression, thereby improving sleep quality; vibration at a frequency of 44 Hz is more conducive to muscle relaxation, eliminating muscle fatigue, restoring muscle strength, and improving muscle tendon elasticity and hardness; vibration at a frequency of 52 Hz helps to relieve lower back pain. In the embodiments of this application, good relaxation effects can be achieved through the single use and combined use of the above vibration modes. After that, the relaxation process in step S250 can help overcome leg fatigue and reduce recovery time. Massage after exercise can relax muscles and relieve fatigue in leg muscles caused by prolonged standing or exercise. It increases local blood and lymph flow, reduces edema, and relieves muscle soreness.

The third-stage relaxation mode first puts the body into a relaxation state through swinging lulling and vibration massage, and then the bed transitions from dynamic to static, reducing interference. In step S310, in a zero-gravity environment, the human body often assumes a specific posture when naturally relaxed and without external force: the angle between the upper torso and the thighs is 128° (±7°), and the angle between the thighs and the calves is 133° (±8°). This posture exerts the least tension and pressure on the nerves, tendons, muscles, and bones. In step S320, slightly elevating the head of the bed can help open the airway and obtain better airflow; it significantly reduces the apnea-hypopnea index (AHI), increases blood oxygen saturation, and improves sleep efficiency.

In some embodiments of this application, the above three stages of the relaxation mode can be completed within 15 minutes. By using the electric bed to help the user relax dynamically before bedtime for 15 minutes, the user can experience three stages: massage relaxation and swinging lulling in a zero-gravity angle state; vibration massage in a horizontal state and vibration massage at an anti-snoring angle; and static sleep aid with the mutual conversion between the zero-gravity and anti-snoring angles, helping the user fall asleep as soon as possible and relieving the user's sleep problems. The function of having the above three stages of the relaxation mode in the device of this application is also referred to as the one-click sleep function in this application.

The applicant, in collaboration with Harbin Institute of Technology, conducted a sleep experiment to verify that the one-click sleep function of the device in this application can help users solve the problem of "insomnia." A total of 100 normal subjects were recruited for this experiment, and each subject was tested twice, once as a control group and once as an experimental group. The two tests for the same subject were spaced apart to avoid continuous testing. The male-to-female ratio was 1:1; the age distribution was evenly split between young and middle-aged groups. The subject screening criteria were: no underlying diseases and no sleep disorders. The equipment used in this experiment included the applicant's smart bed as the passive relaxation apparatus, which integrated the one-click sleep program, a pressure tester, the Dewei EEG acquisition software EegOnline, the Dewei EEG monitoring eye mask NeuroNap, etc.

Experiment environment: dimly lit, 26°C air-conditioned room, no odor, thin blanket available, no direct draft, and silent indoor communication equipment. Note: On the test day, subjects were not allowed to drink tea/coffee or engage in strenuous exercise before collection. Experimental group: 15-minute sleep aid bed program, followed by natural sleep; control group: 15-minute listening to prompt tones and clicking the mouse, followed by natural sleep. Single collection time: about 90 minutes, in the afternoon or early evening.

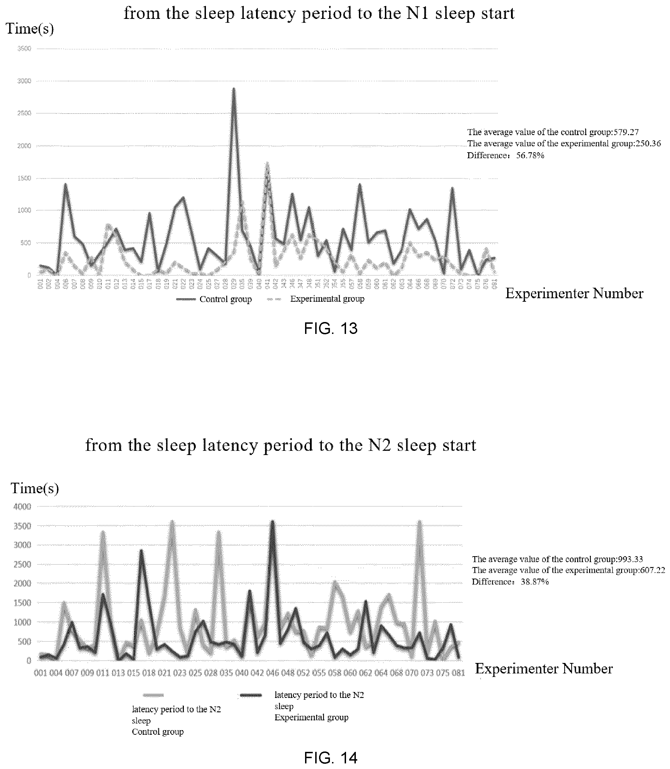

Experiment results: Sleep onset time was defined as the time from turning off the lights to entering the shallow sleep N1 stage, i.e., the sleep latency of the shallow sleep N1 stage. The international AASM sleep staging criteria were used to analyze the characteristics of the signal spectrum and waveform. Among them, the signals of 55 subjects were included in the feature statistical analysis. Based on the currently analyzed data of 55 people, the line charts of sleep latency are shown in Figures 13 and 14: In the experimental group, compared with the control group, the sleep latency in the shallow sleep N1 and shallow sleep N2 stages showed an overall decreasing trend. The sleep latency for entering the shallow sleep N1 stage in the experimental group was shortened by an average of 56.78% compared with the control group. This indicates that after using the one-click sleep function of the device in this application, the sleep onset time in the experimental group was shortened by 56.78%, which is higher than 55%. Therefore, it can be said that the one-click sleep function of the device in this application can shorten the sleep onset time by 55%.

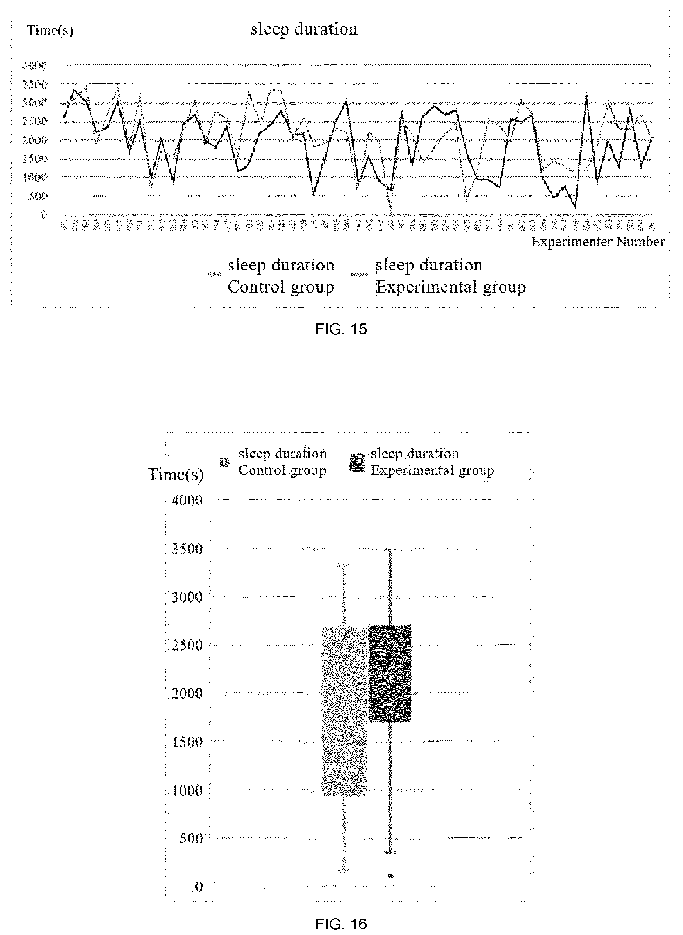

The statistical test results are shown in the line chart in Figure 15, indicating a statistically significant difference (ρ=0.01) in sleep duration between the experimental group and the control group; compared with the control group, the experimental group could extend the sleep duration by an average of 13.43%. Furthermore, the box plot results in Figure 16 show that the overall sleep duration in the experimental group was longer than that in the control group, and the individual sleep durations in the experimental group were more concentrated. In terms of sleep duration and wake time, compared with the control group, the experimental group had a longer sleep duration (ρ=0.01), with an average extension of 13.43%; and a shorter wake time (ρ=0.02). This indicates that after using the one-click sleep function of the device in this application, the experimental group had an increase in sleep time of 13.43% within the same statistical natural sleep time, which is higher than 13%. Therefore, it can be said that the one-click sleep function of the device in this application can extend the sleep time by 13%.

Furthermore, some embodiments of this application also include a report output unit in the passive relaxation apparatus. As shown in Figure 10, the report output unit is configured to output passive relaxation reports in the following two modes, step S400.

The first report output mode is to output a report including the heart rate, respiratory rate, stress index, activity of the autonomic nervous system, fatigue index, and stress resistance after 15 minutes of passive relaxation, step S410.

The second report output mode is to generate an initial test comparison report on the 8th, 15th, and 22nd days after using the passive relaxation apparatus for 7, 14, and 21 days, respectively, and statistically analyze the changes in sleep efficiency, sleep score, deep sleep duration, fatigue index, and recovery index after using this function, step S420.

In one embodiment of this invention, a storage medium is also provided, which stores computer program instructions that are executed according to the sleep aid control method described in any Embodiment.

The storage medium includes permanent and non-permanent, removable and non-removable media, which can implement information storage by any method or technology. Information can be computer-readable instructions, data structures, program modules, or other data. Examples of computer storage media include, but are not limited to, phase-change memory (PRAM), static random-access memory (SRAM), dynamic random-access memory (DRAM), other types of random-access memory (RAM), read-only memory (ROM), electrically erasable programmable read-only memory (EEPROM), flash memory, or other memory technologies, read-only optical discs (CD-ROM), digital versatile discs (DVD), or other optical storage, magnetic cassette tapes, magnetic tape disk storage, or other magnetic storage devices, or any other non-transmission media that can be used to store information accessible by computer equipment.

In one embodiment of this disclosure, a computer device is also provided, including a memory for storing computer program instructions and a processor for executing the computer program instructions. When the computer program instructions are executed by the processor, the computer device is triggered to execute the method described in any Embodiment of this disclosure. In a typical configuration of this disclosure, the computer device includes one or more processors (CPUs), input/output interfaces, network interfaces, and memory.

The memory may include non-permanent storage in computer-readable media, random-access memory (RAM), and/or non-volatile memory, such as read-only memory (ROM) or flash memory (flash RAM). The memory is an example of computer-readable media.

The computer device referred to in this disclosure includes, but is not limited to, any electronic product that can interact with users through a touch panel, such as mobile electronic products like smartphones and tablet computers, which can adopt any operating system, such as the Android operating system or iOS operating system.

## Claims

1. A passive relaxation apparatus, comprising a human body bearing unit (80) and at least one position adjustment component (70); the initial position of the human body bearing unit (80) is horizontal and includes a head support portion (81), a waist support portion (82), and a foot support portion (83) corresponding, in use, to the head position, waist position, and foot position of a human body respectively; the position
adjustment component (70) is configured to change the inclination angle of at least one of said portions of the human body bearing unit (80) according to instructions; the waist support portion (82) further includes a first vibration component (821); the passive relaxation apparatus also includes a control module, which comprises a controller (85) and a relaxation execution unit (841); the controller is configured to generate a first control instruction and a second control instruction in sequence according to the first-stage relaxation mode data; the relaxation execution unit (841) is configured to drive said at least one position adjustment component (70) according to the first control instruction to change the inclination angle of at least one portion of the human body bearing unit (80) and, during this process, drive the first vibration component (821) to operate for a first time period; and drive the position adjustment component to alternately raise the head support portion (81) and the foot support portion (83) according to the second control instruction to form a swinging motion, and perform said swinging motion multiple times; the swinging motion includes driving the head support portion (81) from a horizontal position to a position at a 15-degree angle to the horizontal to raise the head support portion (81) while lowering the foot support portion (83) to a position at a 15-degree angle to the horizontal during the swinging motion's first time period, and driving the foot support portion (83) from a position at a 15-degree angle to the horizontal to a position at a 35-degree angle to the horizontal while driving the head support portion (81) from a position at a 15-degree angle to the horizontal to a position at a 2-degree angle to the horizontal during the swinging motion's second time period;
during each swinging motion, the movement rates of the head support portion (81) and the foot support portion (83) are the same; wherein, the movement speeds of the head support portion (81) during the swinging motion are all the same, and the movement speeds of the foot support portion (83) during the swinging motion are all the same; or, the movement speed of the head support portion (81) during the swinging motion gradually decreases, and the movement speed of the foot support portion (83) during the swinging motion gradually decreases.

2. The passive relaxation apparatus according to claim 1, **characterized in that**: changing the inclination angle of at least one portion of the human body bearing unit (80) according to the first control instruction includes driving the head support portion (81) from a horizontal position to a position at a 15-degree angle to the horizontal, driving the foot support portion (83) from a horizontal position to a position at a 35-degree angle to the horizontal, turning on the first vibration component (821) and maintaining it for the first time period, wherein the first vibration component vibrates at a first intensity during the first time period.

3. The passive relaxation apparatus according to claim 1, **characterized in that**: the swinging motion is performed four times.

4. The passive relaxation apparatus according to claim 1, **characterized in that**: in the human body bearing unit (80), the foot support portion (83) further includes a second vibration component (831); wherein the first vibration component (821), the second vibration component (831), and the position adjustment component (70) are configured to be driven according to instructions of a second-stage relaxation mode.

5. The passive relaxation apparatus according to claim 4, **characterized in that**: the actions instructed by the second-stage relaxation mode include a first control instruction to keep the head support portion (81) and the foot support portion (83) horizontal, turn on the first vibration component (821) and the second vibration component (831), and perform a whole-body vibration massage relaxation with a continuous vibration of 28 Hz to 52 Hz; wherein the first vibration component (821) is configured to continuously vibrate for a second time period at a second intensity, and the second vibration component (831) is configured to continuously vibrate for the second time period at a third intensity for whole-body vibration massage relaxation.

6. The passive relaxation apparatus according to claim 4, **characterized in that**: the second-stage relaxation mode may include any one alone or any two in any order, any three in any order, or all four in any order of a first vibration mode, a second vibration mode, a third vibration mode, and a fourth vibration mode; wherein the first vibration mode includes vibrating at a frequency of 28 Hz; the second vibration mode includes vibrating at a frequency of 40 Hz; the third vibration mode includes vibrating at a frequency of 44 Hz; the fourth vibration mode includes vibrating at a frequency of 52 Hz.

7. The passive relaxation apparatus according to claim 6, **characterized in that**: the control module further includes a mode selection unit (842), which is configured to receive the selection of any one vibration mode or any combination thereof in the second-stage relaxation mode of the passive relaxation apparatus.

8. The passive relaxation apparatus according to claim 7, **characterized in that**: the mode selection unit (842) is configured to determine the vibration mode in the second-stage relaxation mode according to user input.

9. The passive relaxation apparatus according to claim 5, **characterized in that**: the second-stage relaxation mode further includes a fourth control instruction; after the fourth control instruction is executed, the head support portion (81) is raised to 15 degrees relative to a horizontal plane, the foot support portion (83) is placed horizontally, and the second vibration component (831) of the foot support portion is turned on at a fourth intensity for a third time period; then the head support portion (81) moves from 15 degrees relative to the horizontal plane to a horizontal position, and the foot support portion (83) remains horizontal.

10. The passive relaxation apparatus according to claim 4, **characterized in that**: the first vibration component (821), the second vibration component (831), and the position adjustment component (70) are configured to be driven according to instructions in the third-stage relaxation mode.

11. The passive relaxation apparatus according to claim 10, **characterized in that**: the instructions in the third-stage relaxation mode include driving the head support portion (81) to be raised to a position at a 15-degree angle to the horizontal and driving the foot support portion (83) to be raised to a position at a 35-degree angle to the horizontal, and maintaining for a fourth time period; driving the foot support portion (83) to descend to the horizontal and maintaining for a fifth time period; and finally placing the head support portion (81) at the horizontal.

12. The passive relaxation apparatus according to claim 10, **characterized in that**: further comprising a communication unit, which receives the first-stage relaxation mode, the second-stage relaxation mode, and the third-stage relaxation mode sent through a cloud server and transmits parameter adjustment instructions, and sends them to the control module.

## Patentansprüche

1. Vorrichtung zur passiven Entspannung, umfassend ein einen menschlichen Körper tragende Einheit (80) und mindestens eine Positionsverstellungskomponente (70); die Anfangsposition der einen menschlichen Körper tragenden Einheit (80) ist horizontal und enthält einen Kopfstützabschnitt (81), einen Taillenstützabschnitt (82) und einen Fußstützabschnitt (83), die bei Gebrauch jeweils der Kopf-, Taillen- und Fußposition eines menschlichen Körpers entsprechen; die Positionsverstellungskomponente (70) ist so konfiguriert, dass sie den Neigungswinkel von mindestens einem der Abschnitt der einen menschlichen Körper tragenden Einheit (80) gemäß Anweisungen verändert; der Taillenstützabschnitt (82) enthält ferner eine erste Vibrationskomponente (821); die Vorrichtung zur passiven Entspannung enthält außerdem ein Steuerungsmodul, das einen Controller (85) und eine Entspannungs-Ausführungseinheit (841) umfasst; der Controller ist so konfiguriert, dass er gemäß den Entspannungsmodus-Daten der ersten Stufe nacheinander eine erste Steueranweisung und eine zweite Steueranweisung erzeugt; die Entspannungs-Ausführungseinheit (841) ist so konfiguriert, dass sie die mindestens eine Positionsverstellungskomponente (70) gemäß der ersten Steueranweisung ansteuert, um den Neigungswinkel von mindestens einem Abschnitt der einen menschlichen Körper tragenden Einheit (80) zu verändern, und während dieses Vorgangs die erste Vibrationskomponente (821) so ansteuert, dass diese für eine erste Zeitspanne in Betrieb ist; und die Positionsverstellungskomponente so ansteuert, dass sie gemäß der zweiten Steueranweisung abwechselnd den Kopfstützabschnitt (81) und den Fußstützabschnitt (83) anhebt, um eine Schwingbewegung zu erzeugen, und die Schwingbewegung mehrfach ausführt; die Schwingbewegung umfasst das Antreiben des Kopfstützabschnitts (81) von einer horizontalen Position zu einer Position in einem Winkel von 15 Grad zur Horizontalen, um den Kopfstützabschnitt (81) anzuheben, während der Fußstützabschnitt (83) während der ersten Zeitspanne der Schwingbewegung auf eine Position mit einem Winkel von 15 Grad zur Horizontalen abgesenkt wird, und das Antreiben des Fußstützabschnitts (83) von einer Position mit einem Winkel von 15 Grad zur Horizontalen zu einer Position mit einem Winkel von 35 Grad zur Horizontalen, während gleichzeitig der Kopfstützabschnitt (81) von einer Position mit einem Winkel von 15 Grad zur Horizontalen zu einer Position mit einem Winkel von 2 Grad zur Horizontalen während der zweiten Zeitspanne der Schwingbewegung bewegt wird;
während jeder Schwingbewegung sind die Bewegungsgeschwindigkeiten des Kopfstützabschnitts (81) und des Fußstützabschnitts (83) gleich; wobei die Bewegungsgeschwindigkeiten des Kopfstützabschnitts (81) während der Schwingbewegung alle gleich sind und die Bewegungsgeschwindigkeiten des Fußstützabschnitts (83) während der Schwingbewegung alle gleich sind; oder die Bewegungsgeschwindigkeit des Kopfstützabschnitts (81) während der Schwingbewegung allmählich abnimmt und die Bewegungsgeschwindigkeit des Fußstützabschnitts (83) während der Schwingbewegung allmählich abnimmt.

2. Vorrichtung zur passiven Entspannung nach Anspruch 1, **dadurch gekennzeichnet, dass**: das Ändern des Neigungswinkels mindestens eines Abschnitts der einen menschlichen Körper tragenden Einheit (80) gemäß der ersten Steueranweisung das Antreiben des Kopfstützabschnitts (81) von einer horizontalen Position in eine Position mit einem Winkel von 15 Grad zur Horizontalen, das Antreiben des Fußstützabschnitts (83) von einer horizontalen Position in eine Position mit einem Winkel von 35 Grad zur Horizontalen, das Einschalten der ersten Vibrationskomponente (821) und das Aufrechterhalten dieser für die erste Zeitspanne enthält, wobei die erste Vibrationskomponente während der ersten Zeitspanne mit einer ersten Intensität vibriert.

3. Vorrichtung zur passiven Entspannung nach Anspruch 1, **dadurch gekennzeichnet, dass** die Schwingbewegung viermal ausgeführt wird.

4. Vorrichtung zur passiven Entspannung nach Anspruch 1, **dadurch gekennzeichnet, dass**: in der einen menschlichen Körper tragenden Einheit (80) der Fußstützabschnitt (83) ferner eine zweite Vibrationskomponente (831) enthält; wobei die erste Vibrationskomponente (821), die zweite Vibrationskomponente (831) und die Positionsverstellungskomponente (70) so konfiguriert sind, dass sie gemäß den Anweisungen eines Entspannungsmodus der zweiten Stufe angetrieben werden können.

5. Vorrichtung zur passiven Entspannung nach Anspruch 4, **dadurch gekennzeichnet, dass**: die durch den Entspannungsmodus der zweiten Stufe angewiesenen Aktionen eine erste Steueranweisung enthalten, um den Kopfstützabschnitt (81) und den Fußstützabschnitt (83) horizontal zu halten, die erste Vibrationskomponente (821) und die zweite Vibrationskomponente (831) einzuschalten und eine Ganzkörper-Vibrationsmassageentspannung mit einer kontinuierlichen Vibration von 28 Hz bis 52 Hz durchzuführen; wobei die erste Vibrationskomponente (821) so konfiguriert ist, dass sie für eine zweite Zeitspanne mit einer zweiten Intensität kontinuierlich vibriert, und die zweite Vibrationskomponente (831) so konfiguriert ist, dass sie für die zweite Zeitspanne mit einer dritten Intensität kontinuierlich vibriert, um eine Ganzkörper-Vibrationsmassageentspannung durchzuführen.

6. Vorrichtung zur passiven Entspannung nach Anspruch 4, **dadurch gekennzeichnet, dass**: der Entspannungsmodus der zweiten Stufe entweder einen einzelnen oder zwei in beliebiger Reihenfolge, drei in beliebiger Reihenfolge oder alle vier in beliebiger Reihenfolge eines ersten Vibrationsmodus, eines zweiten Vibrationsmodus, eines dritten Vibrationsmodus und eines vierten Vibrationsmodus enthalten kann; wobei der erste Vibrationsmodus Vibrationen mit einer Frequenz von 28 Hz enthält; der zweite Vibrationsmodus Vibrationen mit einer Frequenz von 40 Hz enthält; der dritte Vibrationsmodus Vibrationen mit einer Frequenz von 44 Hz enthält; und der vierte Vibrationsmodus Vibrationen mit einer Frequenz von 52 Hz enthält.

7. Vorrichtung zur passiven Entspannung nach Anspruch 6, **dadurch gekennzeichnet, dass**: das Steuerungsmodul ferner eine Modus-Auswahleinheit (842) enthält, die so konfiguriert ist, dass sie die Auswahl eines beliebigen Vibrationsmodus oder einer beliebigen Kombination davon in dem Entspannungsmodus der zweiten Stufe der Vorrichtung zur passiven Entspannung empfängt.

8. Vorrichtung zur passiven Entspannung nach Anspruch 7, **dadurch gekennzeichnet, dass** die Modus-Auswahleinheit (842) so konfiguriert ist, dass sie den Vibrationsmodus in dem Entspannungsmodus der zweiten Stufe entsprechend der Benutzereingabe bestimmt.

9. Vorrichtung zur passiven Entspannung nach Anspruch 5, **dadurch gekennzeichnet, dass**: der Entspannungsmodus der zweiten Stufe ferner eine vierte Steueranweisung enthält; nach Ausführung der vierten Steueranweisung der Kopfstützabschnitt (81) um 15 Grad gegenüber einer Horizontalebene angehoben wird, der Fußstützabschnitt (83) horizontal positioniert wird, und die zweite Vibrationskomponente (831) des Fußstützabschnitts mit einer vierten Intensität für eine dritte Zeitspanne eingeschaltet wird; anschließend bewegt sich der Kopfstützabschnitt (81) von 15 Grad gegenüber der Horizontalebene in eine horizontale Position, während der Fußstützabschnitt (83) horizontal bleibt.

10. Vorrichtung zur passiven Entspannung nach Anspruch 4, **dadurch gekennzeichnet, dass**: die erste Vibrationskomponente (821), die zweite Vibrationskomponente (831) und die Positionsverstellungskomponente (70) so konfiguriert sind, dass sie gemäß den Anweisungen in dem Entspannungsmodus der dritten Stufe angetrieben werden können.

11. Vorrichtung zur passiven Entspannung nach Anspruch 10, **dadurch gekennzeichnet, dass**: die Anweisungen in dem Entspannungsmodus der dritten Stufe das Antreiben des Kopfstützabschnitts (81), so dass er in eine Position mit einem Winkel von 15 Grad zur Horizontalen angehoben wird, und das Antreiben des Fußstützabschnitts (83), so dass er in eine Position mit einem Winkel von 35 Grad zur Horizontalen angehoben wird, und das Aufrechterhalten für eine vierte Zeitspanne; das Antreiben des Fußstützabschnitts (83), um auf die Horizontale abgesenkt zu werden, und das Aufrechterhalten für eine fünfte Zeitspanne; und schließlich das Platzieren des Kopfstützabschnitts (81) in die Horizontalen enthält.

12. Vorrichtung zur passiven Entspannung nach Anspruch 10, **dadurch gekennzeichnet, dass** sie ferner eine Kommunikationseinheit umfasst, die den über einen Cloud-Server gesendeten Entspannungsmodus der ersten Stufe, den Entspannungsmodus der zweiten Stufe und den Entspannungsmodus der dritten Stufe empfängt und Parameteranpassungsanweisungen überträgt und diese an das Steuerungsmodul sendet.

## Revendications

1. Appareil de relaxation passive, comportant une unité de support de corps humain (80) et au moins un composant de réglage de position (70) ; la position initiale de l'unité de support de corps humain (80) est horizontale et comporte une partie de soutien de tête (81), une partie de soutien de taille (82) et une partie de soutien de pieds (83) correspondant respectivement, en cours d'utilisation, à la position de la tête, à la position de la taille et à la position des pieds d'un corps humain ; le composant de réglage de position (70) est configuré pour modifier l'angle d'inclinaison d'au moins l'une desdites parties de l'unité de support de corps humain (80) selon des instructions ; la partie de soutien de taille (82) comporte en outre un premier composant de vibration (821) ; l'appareil de relaxation passive comporte également un module de commande, qui comporte un dispositif de commande (85) et une unité d'exécution de relaxation (841) ; le dispositif de commande est configuré pour générer une première instruction de commande et une deuxième instruction de commande en séquence selon les données de mode de relaxation de premier étage ; l'unité d'exécution de relaxation (841) est configurée pour entraîner ledit au moins un composant de réglage de position (70) selon la première instruction de commande pour modifier l'angle d'inclinaison d'au moins une partie de l'unité de support de corps humain (80) et, pendant ce processus, entraîner le fonctionnement du premier composant de vibration (821) pendant une première période de temps ; et entraîner le composant de réglage de position pour relever alternativement la partie de soutien de tête (81) et la partie de soutien de pieds (83) selon la deuxième instruction de commande pour former un mouvement de balancement, et réaliser ledit mouvement de balancement plusieurs fois ; le mouvement de balancement comprend l'entraînement de la partie de soutien de tête (81) d'une position horizontale à une position à un angle de 15 degrés par rapport à l'horizontale pour relever la partie de soutien de tête (81) tout en abaissant la partie de soutien de pieds (83) à une position à un angle de 15 degrés par rapport à l'horizontale pendant la première période de temps du mouvement de balancement, et l'entraînement de la partie de soutien de pieds (83) d'une position à un angle de 15 degrés par rapport à l'horizontale à une position à un angle de 35 degrés par rapport à l'horizontale tout en amenant la partie de soutien de tête (81) d'une position à un angle de 15 degrés par rapport à l'horizontale à une position à un angle de 2 degrés par rapport à l'horizontale pendant la deuxième période de temps du mouvement de balancement ;
pendant chaque mouvement de balancement, les vitesses de mouvement de la partie de soutien de tête (81) et de la partie de soutien de pieds (83) sont identiques ; dans lequel les vitesses de mouvement de la partie de soutien de tête (81) pendant le mouvement de balancement sont toutes identiques, et les vitesses de mouvement de la partie de soutien de pieds (83) pendant le mouvement de balancement sont toutes identiques ; ou la vitesse de mouvement de la partie de soutien de tête (81) pendant le mouvement de balancement diminue progressivement, et la vitesse de mouvement de la partie de soutien de pieds (83) pendant le mouvement de balancement diminue progressivement.

2. Appareil de relaxation passive selon la revendication 1, **caractérisé en ce que** : la modification de l'angle d'inclinaison d'au moins une partie de l'unité de support de corps humain (80) selon la première instruction de commande comprend l'entraînement de la partie de soutien de tête (81) d'une position horizontale à une position à un angle de 15 degrés par rapport à l'horizontale, l'entraînement de la partie de soutien de pieds (83) d'une position horizontale à une position à un angle de 35 degrés par rapport à l'horizontale, la mise en marche du premier composant de vibration (821) et son maintien pendant la première période de temps, dans lequel le premier composant de vibration vibre à une première intensité pendant la première période de temps.

3. Appareil de relaxation passive selon la revendication 1, **caractérisé en ce que** : le mouvement de balancement est réalisé quatre fois.

4. Appareil de relaxation passive selon la revendication 1, **caractérisé en ce que** : dans l'unité de support de corps humain (80), la partie de soutien de pieds (83) comporte en outre un deuxième composant de vibration (831) ; dans lequel le premier composant de vibration (821), le deuxième composant de vibration (831) et le composant de réglage de position (70) sont configurés pour être entraînés selon des instructions d'un mode de relaxation de deuxième étage.

5. Appareil de relaxation passive selon la revendication 4, **caractérisé en ce que** : les actions demandées par le mode de relaxation de deuxième étage comportent une première instruction de commande pour maintenir la partie de soutien de tête (81) et la partie de soutien de pieds (83) horizontales, mettre en marche le premier composant de vibration (821) et le deuxième composant de vibration (831), et réaliser un massage par vibration du corps entier avec une vibration continue de 28 Hz à 52 Hz ; dans lequel le premier composant de vibration (821) est configuré pour vibrer en continu pendant une deuxième période de temps à une deuxième intensité, et le deuxième composant de vibration (831) est configuré pour vibrer en continu pendant la deuxième période de temps à une troisième intensité pour une relaxation par massage par vibration du corps entier.

6. Appareil de relaxation passive selon la revendication 4, **caractérisé en ce que** : le mode de relaxation de deuxième étage peut comprendre un seul ou deux dans n'importe quel ordre, trois dans n'importe quel ordre, ou les quatre dans n'importe quel ordre d'un premier mode de vibration, d'un deuxième mode de vibration, d'un troisième mode de vibration et d'un quatrième mode de vibration ; dans lequel le premier mode de vibration comporte une vibration à une fréquence de 28 Hz ; le deuxième mode de vibration comporte une vibration à une fréquence de 40 Hz ; le troisième mode de vibration comporte une vibration à une fréquence de 44 Hz ; le quatrième mode de vibration comporte une vibration à une fréquence de 52 Hz.

7. Appareil de relaxation passive selon la revendication 6, **caractérisé en ce que** : le module de commande comporte en outre une unité de sélection de mode (842), qui est configurée pour recevoir la sélection d'un mode de vibration quelconque ou de toute combinaison de celui-ci dans le mode de relaxation de deuxième étage de l'appareil de relaxation passive.

8. Appareil de relaxation passive selon la revendication 7, **caractérisé en ce que** : l'unité de sélection de mode (842) est configurée pour déterminer le mode de vibration dans le mode de relaxation de deuxième étage selon une entrée utilisateur.

9. Appareil de relaxation passive selon la revendication 5, **caractérisé en ce que** : le mode de relaxation de deuxième étage comporte en outre une quatrième instruction de commande ; après l'exécution de la quatrième instruction de commande, la partie de soutien de tête (81) est relevée à 15 degrés par rapport à un plan horizontal, la partie de soutien de pieds (83) est placée horizontalement, et le deuxième composant de vibration (831) de la partie de soutien de pieds est activé à une quatrième intensité pendant une troisième période de temps ; puis la partie de soutien de tête (81) se déplace de 15 degrés par rapport au plan horizontal vers une position horizontale, et la partie de soutien de pieds (83) reste horizontale.

10. Appareil de relaxation passive selon la revendication 4, **caractérisé en ce que** : le premier composant de vibration (821), le deuxième composant de vibration (831) et le composant de réglage de position (70) sont configurés pour être entraînés selon des instructions dans le mode de relaxation de troisième étage.

11. Appareil de relaxation passive selon la revendication 10, **caractérisé en ce que** : les instructions dans le mode de relaxation de troisième étage comprennent l'entraînement de la partie de soutien de tête (81) pour qu'elle soit relevée dans une position à un angle de 15 degrés par rapport à l'horizontale et l'entraînement de la partie de soutien de pieds (83) pour qu'elle soit relevée dans une position à un angle de 35 degrés par rapport à l'horizontale, et leur maintien pendant une quatrième période de temps ; l'entraînement de la partie de soutien de pieds (83) pour qu'elle descende à l'horizontale et son maintien pendant une cinquième période de temps ; et enfin la mise en place de la partie de soutien de tête (81) à l'horizontale.

12. Appareil de relaxation passive selon la revendication 10, **caractérisé en ce que** : il comporte en outre une unité de communication, qui reçoit le mode de relaxation de premier étage, le mode de relaxation de deuxième étage et le mode de relaxation de troisième étage envoyés par l'intermédiaire d'un serveur en nuage et transmet des instructions d'ajustement de paramètres, et les envoie au module de commande.
